# EUROPEAN PATENT APPLICATION

(11) **EP 2 546 244 A1**
(43) Date of publication of application: **16.01.2013**
(21) Application number: 11173870.4
(22) Date of filing: 13.07.2011
(51) Int. Cl.: C07D 307/92, C07C 35/23

(54) **Supercritical process for manufacturing ambradiol, sclareolide and (-)-ambrafuran from sclareol**

(71) Applicant: Koste Biochemicals, 75015 Paris (FR)
(72) Inventor: Carey, Charles, 75015 Paris (FR)
(74) Representative: Icosa

(57) **Abstract**

The present invention relates to a process for manufacturing products of interest selected from the group comprising ambradiol, sclareolide and (-)-ambrafuran, from a starting material which is sclareol, said process comprising a step of biological conversion of sclareol and at least one further step performed within a pressured vessel, which preferably is a purification step using CO₂ supercritical extraction, or a further chemical conversion step which is selected from the group comprising supercritical hydrogenation, supercritical dehydration, hydrogenation in subcritical water or dehydration in subcritical water.

## Description

### FIELD OF INVENTION

The present invention relates to a process for manufacturing products of interest selected from the group comprising ambradiol, sclareolide and (-)-ambrafuran, from a starting material which is sclareol. More specifically, the present invention pertains to a process comprising a step of biological conversion of sclareol and at least a further step performed within a pressured vessel leading to products of interest. The step performed within a pressure vessel may be a purification step comprising a supercritical extraction or a conversion step such as, but not limited to, a hydrogenation or a dehydration.

### BACKGROUND OF INVENTION

Dodecahydro-3a,6,6,9a-tetramethylnaphto[2,1-b]furan, also called ambrafuran and marketed by Firmenich S.A. under the trademark Ambrox®, is an important fragrance chemical. This molecule, and especially (-)-ambrafuran, its Laevo isomer (3aR,5aR,9aS,9bS)-3a,6,6,9a-tetramethyl-dodecahydronaphto[2,1-b]furan, is responsible for the characteristic odor of ambergris - a naturally occurring amber note with a rich woody character - and is used as a fixative agent in perfumes. This fragrance is used in perfumery but also increasingly in hygiene products or detergents. The production of ambrafuran is estimated to about 20 tonnes/year.

Ambergris is a metabolic substance produced in the digestive track of the sperm whale. When expelled from the whale, ambergris floats at the surface of the sea, being exposed to air and sunlight. These conditions led to changes in the substance and are essential to the development of the fragrance. Due to its origin, natural ambergris is extremely rare. Moreover, its commercial exploitation has been forbidden by the Washington Treaty. Due to the value of ambergris, extensive research programs have been conducted to analyze its composition, revealing the importance of ambrafuran and especially its (-)-ambrafuran isomer. Several routes of chemical synthesis of (-)-ambrafuran were explored. Total syntheses were developed as well as hemi-syntheses, starting from naturally occurring sesqui- or di-terpens such as sclareol, a compound extracted from *Salvia sclarea* (clary sage).

The main drawback of these syntheses is the high number of steps needed to obtain ambrafuran, usually eight steps from sclareol as reported in US patent application US2010/0248316. The last steps of the chemical synthesis are reported below, showing the synthesis of the intermediate products sclareolide and ambradiol:

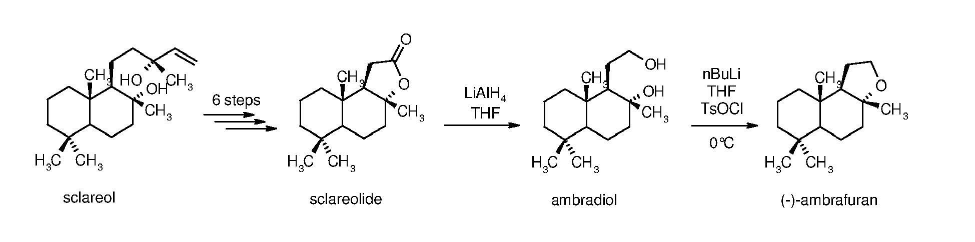

Chemical syntheses also imply the use of harsh conditions for certain steps (corrosive compounds, low temperature, toxic and dangerous reactants) that would be preferably avoided. Also, the use of organic solvents in chemical syntheses presents several drawbacks such as: drying costs, potential toxicity for consumers of solvent residues in the final product, lack of solvent selectivity resulting in the presence of unwanted compounds in the final product. Another concern with chemical syntheses of ambrafuran is that they generally result into racemic mixtures instead of isolated enantiomers. Moreover, consumers are more and more asking for "green products" obtained by environmentally friendly processes compatible with sustainable growth. One of the objectives of sustainable growth consists in avoiding the use of chemical reagents and the rejection of residual salts in the environment, which is incompatible with conventional syntheses of ambrafuran.

An alternative to chemical hemi-synthesis of ambrafuran from sclareol consists in replacing the first six or seven steps of the traditional transformation process leading respectively to sclareolide and to ambradiol intermediates with a biological conversion step.

The biological conversion of sclareol to sclareolide and ambradiol was first reported in patent US4,798,799 for the synthesis of ambradiol and in patent US4,970,163 for the synthesis of sclareolide and ambradiol.

The biological conversion of sclareol to sclareolide described in patent US4,970,163 uses the microorganisms *Cryptococcus albidus* saito, skinner var. albidus, ATCC 20918 or *Cryptococcus albidus,* ATCC 20921. Such biotransformation is carried out under aerobic conditions, in an aqueous nutrient medium containing sclareol. Further isolation and purification of sclareolide may be achieved by conventional techniques such as filtration or centrifugation, by solvent extraction, distillation or crystallization. A method for purifying sclareolide is reported in US patent US5,945,546. Once extracted and purified from the fermentation broth, sclareolide may then be converted into ambradiol by hydrogenation as reported in the eight-step chemical synthesis.

The biological conversion of sclareol to ambradiol is described in patent US4,798,799 using the microorganism *Hyphozyma roseoniger* (CBS214.83 and ATCC 20624) and in patent US4,970,163 using the microorganisms Bensingtonia Ciliata ATCC 20919 or Cryptococcus Laurentii ATCC 20920. Such biotransformations are carried out under aerobic conditions at 20°C, in an aqueous nutrient medium containing sclareol.

In patent US4,798,799, ambradiol is then dehydrated to give ambrafuran directly from the aqueous nutrient medium or after recovery. More specifically, recovery and purification of ambradiol may be achieved by conventional techniques such as filtration or centrifugation, by solvent extraction, distillation or crystallization. Especially, ambradiol may be extracted with ethyl acetate and crystallized from hexane/chloroform.

The conversion of ambradiol to (-)-ambrafuran may then be performed by conventional cyclization methods, such as by reacting ambradiol with toluene-p-sulfonylchloride in pyridine at 0°C, followed by solvent extraction. Alternatively, dehydration is achieved in the presence of an acid catalyst, most often in the form of a strong mineral acid, heteropolyacids, sulfonic acids or DMSO. One of the drawbacks of such methods is that in acidic conditions, ambrafuran is likely to isomerize readily to the more thermodynamically stable, but olfactively much weaker iso-ambrafuran. Also, such methods do not solve the problems associated with chemical synthesis as they also contribute to undesired environmental and economic burdens through the rejection of waste salts, the need to regenerate the catalyst or the use of organic petrochemical solvents.

Patent application US2010/0248316 proposes an alternative cyclization method consisting in exposing ambradiol to an activated zeolite at a temperature between 0°C and 110°C for a period of between 1 and 24 hours. This method allows the dehydration of the diol into (-)-ambrafuran, ambradiol being obtained by the biological conversion disclosed in US4,798,799. Therefore, starting from racemic natural sclareol, this enantioselective method achieves the production of a single enantiomer of ambrafuran in two steps.

In patent application US2010/0248316, the dehydration of ambradiol on activated zeolite is carried out in hexane or in toluene at room temperature or in diméthylsulfoxyde or ethyl acetate, optionally heating the solution. At the end of the reaction, solvent should be removed under reduced pressure.

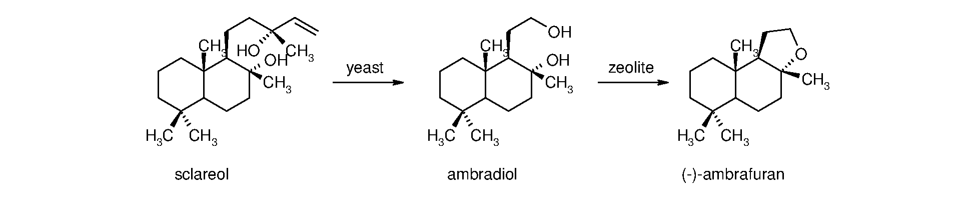

Zeolites are aluminosilicates which are well known as solid acid catalysts. The main features of these materials are their channel dimensions and stable structures. Their use in industrial processes generally results in a reduction in waste and pollution. Contrary to other dehydrations that need controlled temperatures, patent application US2010/0248316 discloses a total conversion at room temperature, generally in less than two hours. The zeolite used is a Group II A metal zeolite, with calcium as metal.

Zeolites need to be activated before being used as catalysts, especially by removing from active sites water or other small molecules that may have been adsorbed. In US2010/0248316, one treatment consists in a reflux treatment at 90°C for 24 hours in presence of ammonium nitrate. Another treatment is activation at 500°C under vacuum or with a conventional microwave oven. After treatment, the activated zeolite should be kept in a closed container before use to avoid inactivation through contact with ambient air.

Despite advantages of the method disclosed in US2010/0248316 such as the reduction of the number of steps or the suppression of toxic reactants, several drawbacks remain. One of them is the use of specific organic solvents to extract and purify ambradiol from the fermentation broth, to carry out the dehydration reaction and to extract and purify (-)-ambrafuran. Specifically, during the dehydration step, only toluene and hexane are claimed to afford 100% conversion rate in 4 hours at room temperature while ethanol or ethyl acetate afford conversion rates of only 5.4% and 3.7% respectively. The costs of solvents, the manipulations needed as well as the evaporation steps are not optimal. Moreover, solvent use often results in atmospheric contamination during drying steps or disposal of spent material and may contaminate the final product, which is not environmentally friendly, requires costly recycling processes and may constitute health risks for consumers. Another drawback is the restrictive activation step of the zeolite. Indeed, the treatment described for zeolite activation rules out the establishment of a fully continuous production process. Moreover, the manipulation of the activated zeolite from the drying site to the dehydration site automatically induces a partial inactivation of the zeolite through air contact, which in turn results in using a higher ratio of zeolite to ambradiol. In order to avoid zeolite inactivation and zeolite recharging, use would have to be performed in controlled airtight atmosphere, which inevitably contributes to generating extra costs compared to a minimal setup.

### TECHNICAL PROBLEM

Therefore, there is a need for an improved process of production of products of interest selected from the group comprising sclareolide, ambradiol and/or (-)-ambrafuran from sclareol presenting the following characteristics:
- avoid the use of synthetic solvents or organic solvents of petrochemical origin at any step of the transformation process, in particular during purification or transformation steps, and/or
- avoid the use of chemical reactants that result in the production of salts and other potentially toxic or polluting spent material, and/or
- use a continuous process to reduce the time of transformation and number of steps involved, and/or
- simplify the conditions of conversion of sclareolide and/or ambradiol leading to the production of (-)-ambrafuran, and/or

Moreover, improvements of purity are expected since even though (-)-ambrafuran is obtainable with a purity of more than 99% from total chemical synthesis, it is obtained at the best with 95% of purity from hemi-synthesis (i.e. starting from vegetal material).

The Applicant found solutions to this technical problem when he began to show that:
● sclareolide, ambradiol and (-)-ambrafuran are soluble in supercritical CO₂ and HTW at the temperature and pressure conditions required for the concerned purifications and chemical transformations,
● these products are stable at the often high temperature and pressure conditions required for the concerned transformations, and
● reactions of hydrogenation and dehydration and combinations thereof can be performed on sclareolide and ambradiol in supercritical fluid and/or HTW conditions.

Especially, in this invention, the Applicant identified a transformation process that can not only achieve the purification of sclareolide and/or ambrafuran from a bioconversion broth and chemical conversion through hydrogenation and dehydration of such substrates into (-)-ambrafuran but offers sufficient flexibility to achieve the same purification from broth and/or types of chemical conversions using other substrates, solvents, reagents, catalysts and/or operating conditions.

The Applicant also found that not only supercritical conditions were useful for the purification of intermediate or final compounds, i.e. sclareolide, ambradiol and (-)-ambrafuran, but also that supercritical fluids or HTW could be used as medium and/or reagent and/or catalyst and/or solvent for the chemical conversion of sclareolide and ambradiol into (-)-ambrafuran.

Also, the use of supercritical fluids and/or HTW as solvents and/or media and/or catalysts and/or reactants along the process allowed the applicant to not only identify a continuous process but also a device capable of implementing continuously the desired chemical transformations on sclareolide and ambradiol, while offering the flexibility to further accommodate the use of various media, solvents, reactants and catalysts in closed loops at different steps of the production process and while further avoiding unnecessary changes of pressure and/or temperature, mixing of reactants, media, solvents and/or catalysts.

### SUMMARY

This invention thus pertains to a process for manufacturing and purifying ambradiol, sclareolide and/or (-)-ambrafuran from a starting material which is sclareol, said process implementing a step performed within a pressured vessel, which is preferably a step under supercritical conditions.

More precisely, this invention pertains to a process for manufacturing and purifying products of interest selected from the group comprising ambradiol, sclareolide and (-)-ambrafuran, from a starting material which is sclareol, said process comprising a step of biological conversion of sclareol and at least one further step performed within a pressured vessel.

In an embodiment, the further step is a purification step using a supercritical fluid, potentially with a cosolvent or a further chemical conversion step selected from the group comprising hydrogenation in a supercritical fluid, dehydration in a supercritical fluid, hydrogenation in subcritical water or dehydration in subcritical water or a combination thereof performed in one step in supercritical fluid and/or subcritical water.

According to an embodiment, the process is continuous or semi-continuous.

In an embodiment, the pressured vessel is coupled to a heating device. In another embodiment, the pressured vessel is equipped with a vacuum pump. Of course, the pressured vessel may be both coupled to a heating device and equipped with a vacuum pump.

In an embodiment, the vessel is charged with a catalyst, which is preferably a metal catalyst on solid support.

In an embodiment, the vessel is charged with a solid desiccant, which is preferably an activated zeolite.

According to the invention, the biological conversion is performed with sclareol as a starting material whereby sclareol is contacted with a microorganism capable of converting it into sclareolide or ambradiol, said microorganism being in an aqueous nutrient medium, and the further step performed within a pressured vessel being at least one chemical transformation, and/or a purification, of sclareolide or ambradiol.

In an embodiment, the microorganism is selected from the group comprising *Cryptococcus albidus* saito, *skinner var. albidus,* ATCC 20918 or *Cryptococcus albidus,* ATCC 20921, and is capable to convert sclareol into sclareolide. Then, sclareolide may be purified within a pressured vessel and recovered, or further transformed into ambradiol, which may be purified within a pressured vessel and recovered or further transformed into (-)-ambrafuran. Alternatively, sclareolide may be transformed directly into (-)-ambrafuran or a mixture of ambradiol and (-)-ambrafuran, which may be purified within a pressure vessel and recovered or further transformed into an even more pure (-)-ambrafuran.

In an alternative embodiment, the microorganism is selected from the group comprising *Hyphozyma roseoniger* (CBS214.83 and ATCC 20624), Bensingtonia Ciliata ATCC 20919 or Cryptococcus Laurentii ATCC 20920 and is capable to convert sclareol into ambradiol, which may be purified within a pressured vessel and recovered or transformed into (-)-ambrafuran.

In an embodiment, sclareolide and/or ambradiol are purified through supercritical CO₂ extraction followed, whenever necessary, by a separation step to recover purified sclareolide or ambradiol from the supercritical fluid.

In an embodiment, sclareolide is chemically transformed into ambradiol through a hydrogenation step within a pressured vessel under supercritical fluid conditions or within a vessel containing HTW.

In an embodiment, the hydrogenation step is catalyzed under high temperature by a metal catalyst or mix thereof on solid support with the addition of gazeous hydrogen under pressure, preferably in the presence of supercritical CO₂.

In an embodiment, the hydrogenation step is catalyzed by HTW, potentially with the addition of a further catalyst which may consist of supercritical CO₂ or other solid catalysts.

In an embodiment, sclareolide is chemically transformed into (-) ambrafuran or a mix of ambradiol and (-) ambrafuran within a pressured vessel under supercritical CO₂ conditions or within a vessel containing HTW.

In an embodiment, the conversion of sclareolide into (-) ambrafuran or a mix of ambradiol and (-) ambrafuran is catalyzed under high temperature by a metal catalyst or mix thereof on solid support with the addition of gazeous hydrogen under pressure, preferably in the presence of supercritical CO₂.

In an embodiment, the conversion of sclareolide into (-) ambrafuran or a mix of ambradiol and (-) ambrafuran is catalyzed by HTW, potentially with the addition of a further catalyst which may consist of supercritical CO₂ or other solid catalysts.

In an embodiment, ambradiol is chemically transformed into (-)-ambrafuran through a dehydration step within a pressured vessel under supercritical CO₂ conditions or within a vessel containing HTW.

In an embodiment, the dehydration is catalyzed by a solid desiccant, preferably an activated zeolite charged in the pressured vessel under supercritical CO₂ conditions.

In an embodiment, the dehydration is catalyzed by a solid desiccant, preferably an activated zeolite catalyst charged in the pressured vessel and the dehydration is performed in supercritical CO₂.

In an embodiment, the dehydration is catalyzed by HTW, potentially with the addition of a further catalyst which may consist of supercritical CO₂ or other solid catalysts.

In an embodiment, the supercritical extraction occurs at a pressure of 80 to 350 bars and preferably a pressure of 80 to 150 bars and at a temperature of 15 to 250°C and preferably at room temperature.

This invention also pertains to a device for implementing a process according to the present invention, comprising a pressured vessel optionally packed with a catalyst, which preferably is HTW or an activated zeolite, the pressured vessel being preferably associated with a heating device capable of heating the walls of the vessel or with an internal microwave device capable of heating the content of the vessel.

### DEFINITIONS

In the present invention, the following terms have the following meanings:
- "**continuous**" refers to a transformation process whereby production can be performed with no interruption assuming a constant supply to the process of the components necessary for the production, such as .substrate to be transformed, power or catalysts. Continuous can also be defined in contrast to batch processes where only a fixed amount of product can be processed at a time, usually in vessels or containers, before some of the elements necessary to the process need to be reloaded, recharged or exchanged through process interruption.
- **"purification"** refers to the action of isolating a chosen compound usually from one or several compounds. For the purpose of this paper, "purification" will include the action generally referred to as "extraction" and consisting of the initial isolation of a compound or mix of compounds containing the targeted compound from a raw material, which can be for example a plant or a bioconversion broth.
- **"supercritical extraction"** refers to the action of extracting a substance from a mix of substances by contacting such mix with a supercritical fluid in a pressured vessel (the extractor) so as to dissolve selectively the targeted substance in the supercritical fluid. The charged supercritical fluid is then transferred to another vessel (the separator) where it is expanded to it gaseous form, thereby isolating the targeted compound.
- **"subcritical water"** and **"HTW"** refers to water at temperatures ranging from 100°C to 390°C.
- **"supercritical CO₂"** refers to CO₂ potentially mixed with a cosolvent such as ethanol at temperatures above 30.95 °C and pressures above 73.8 bar.
- **"hydrogenation",** for the purpose of this paper, consists of the addition of hydrogen atoms to a molecule and will also include reactions of reduction. The transformation of sclareolide into ambradiol will fall within this definition.
- **"dehydration"** refers to the removal of a molecule of water from a substance. The transformation of ambradiol to (-)-ambrafuran will fall within this definition.

### BRIEF DESCRIPTION OF THE DRAWINGS

- **Figure 1** is a scheme showing different routes of synthesis of sclareolide, ambradiol and (-)-ambrafuran comprising an enzymatic step and a further step in a pressured vessel.
- **Figure 2** is a scheme showing a process of synthesis of ambradiol and/or (-)-ambrafuran comprising an enzymatic step and a further conversion step in a pressure-controlled vessel using high-temperature water (HTW).
- **Figure 3-A** is a scheme showing a process of synthesis of ambradiol and/or (-)-ambrafuran comprising an enzymatic step, a supercritical extraction and a further hydrogenation in a pressured vessel.
- **Figure 3-B** is a scheme showing a process of synthesis of (-)-ambrafuran comprising an enzymatic step, a supercritical extraction and a further dehydration in a pressured vessel.
- **Figure 3-C** is a scheme showing a process of synthesis of ambradiol and/or (-)-ambrafuran comprising an enzymatic step, a supercritical extraction and a further conversion step in a pressure-controlled vessel using HTW.
- **Figure 4** is a scheme showing a process of synthesis of (-)-ambrafuran comprising an enzymatic step, a supercritical extraction and two further conversion steps in pressured vessels.
- **Figure 5** is a scheme showing catalytic transformation vessel capable of performing hydrogenations, dehydrations and combinations thereof on a variety af substrates, mediums, catalysts and reactants.

### DETAILED DESCRIPTION

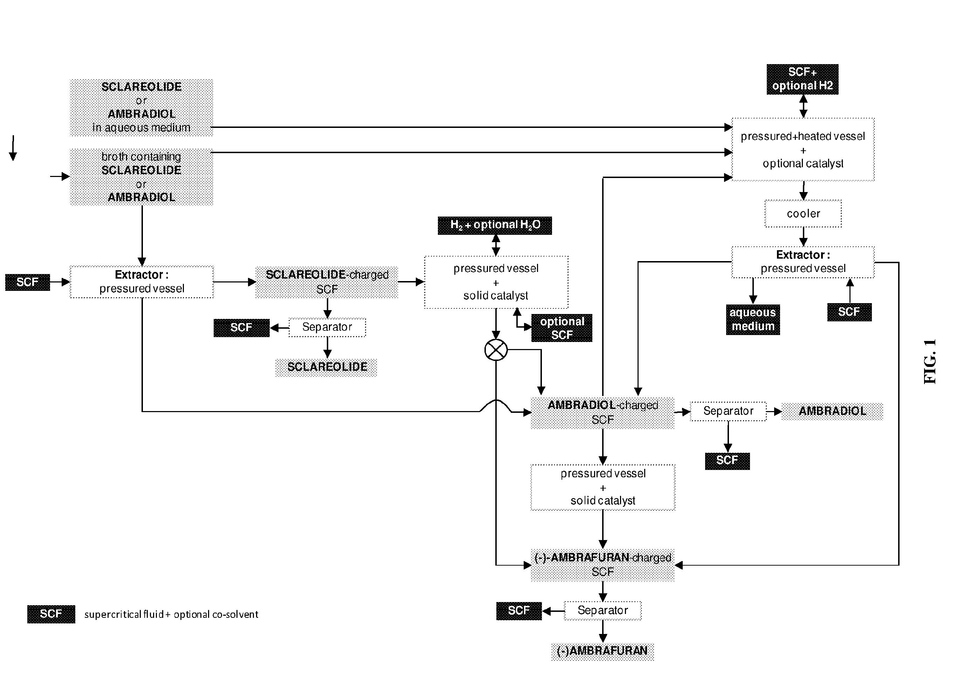

This detailed description may be read with reference to Fig.1.

This invention relates to a process of manufacturing products of interest selected from the group comprising sclareolide, ambradiol and/or (-)-ambrafuran from sclareol, comprising a step of biological conversion and at least one further step, performed within a pressured vessel, which may be a purification step or a further conversion of products or biological conversion into ambradiol and/or (-)-ambrafuran.

### Biological conversion

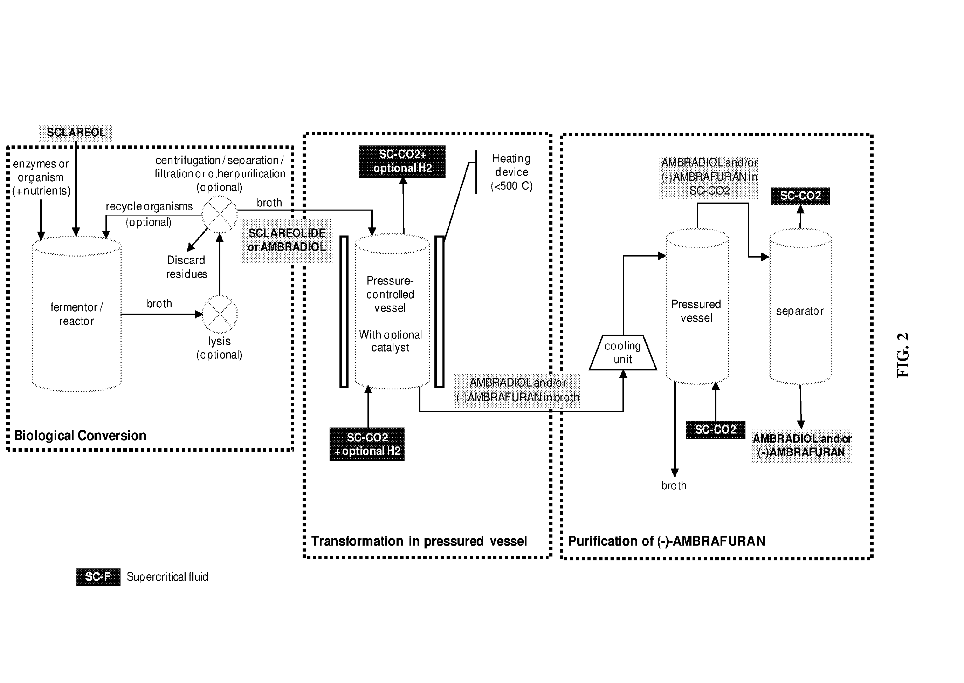

As shown in Fig.1, the first step is a biological conversion step. According to one embodiment, the biological conversion step of the present invention using sclareol as a substrate and using a suitable microorganism, generally within an aqueous nutrient medium, may produce sclareolide or ambradiol. A detailed view of the biological conversion is shown in Fig.2, including possible recycling of yeast.

### First and optional purification

As a preliminary and optional step, the fermentation broth resulting from the biological conversion step and containing sclareolide or ambradiol is purified. Said purification may be performed by means of centrifugation, filtration or separation techniques.

### Further transformation step in pressured vessel

According to one embodiment, as shown in Fig.2, the fermentation broth containing purified or non-purified sclareolide or ambradiol is used in a further conversion step of the process, i.e. further transformation(s) towards ambradiol and/or (-)-ambrafuran by passing it into a pressure-controlled and heated vessel under HTW conditions, such vessel being optionally prefilled with a solid catalyst and/or preferably while feeding supercritical CO₂ or other fluids and/or reactants in gaseous form such as dihydrogen, in a counter-current manner in said vessel.

In an embodiment, the mix of supercritical fluids and gases injected counter-currently in the pressure-controlled vessel is recycled toward the same vessel.

In an embodiment, transformed products are recovered after cooling off the broth and feeding it to a supercritical extractor where a supercritical fluid, preferably supercritical CO2, is fed countercurrently. Transformed products are then recovered by expanding the supercritical fluid in a separator.

In another embodiment, transformed products are recovered by filtration, centrifugation, separation or other methods known to the art.

### Further purification step in pressured vessel, followed by transformation

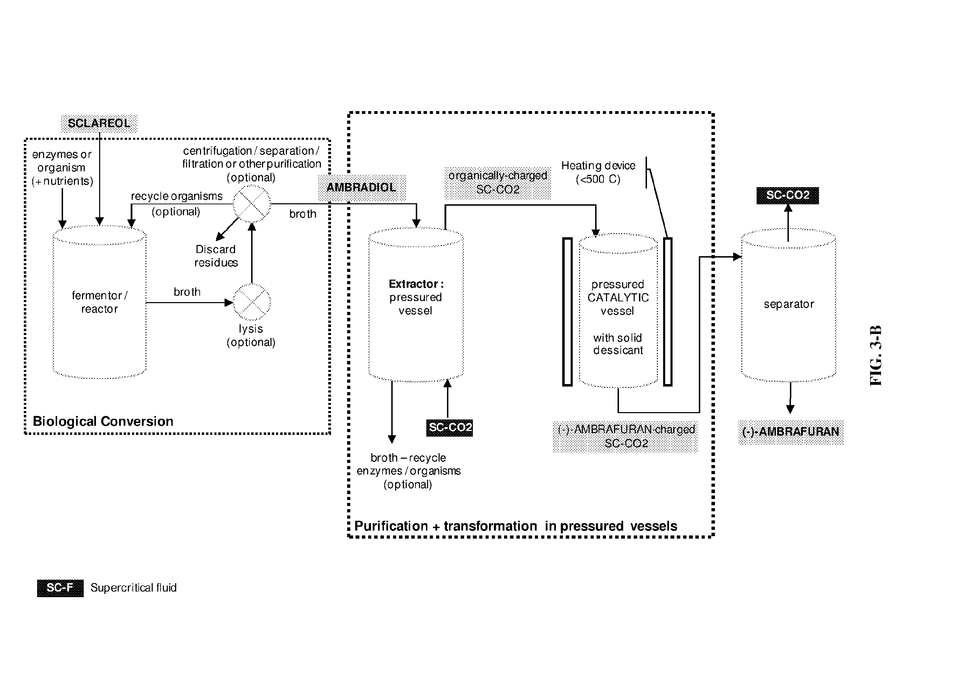

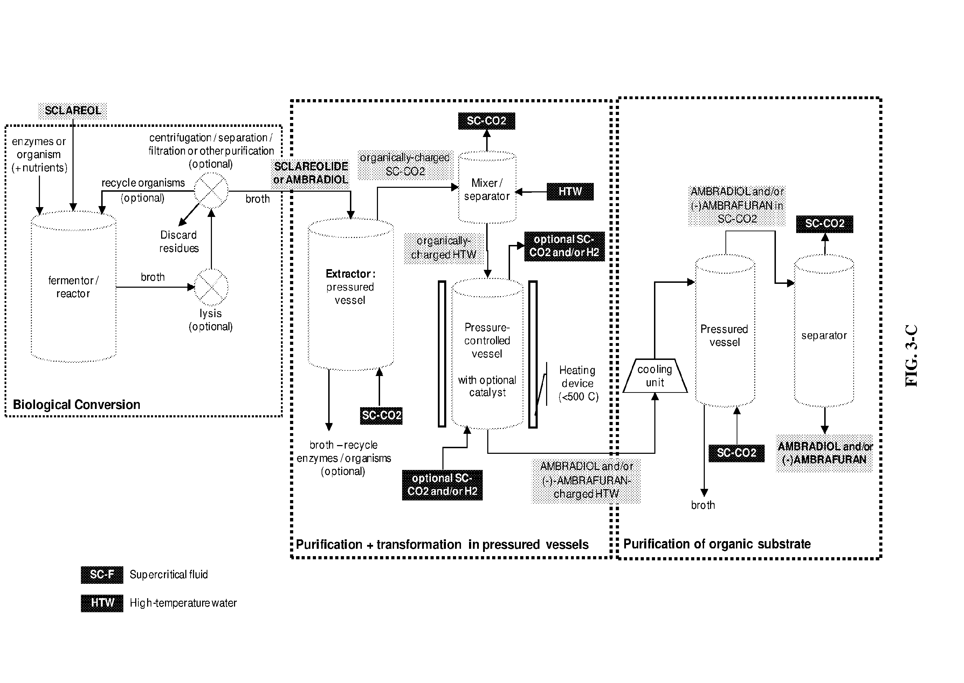

According to another embodiment, as shown in Fig.3-B, 3-B and 3-C, sclareolide or ambradiol is purified using a supercritical fluid to recover pure sclareolide or pure ambradiol.

In these embodiments, the purified ambradiol and/or sclareolide may further be converted into ambradiol and/or (-)-ambrafuran by passing it into a pressured vessel, optionally heated and preferably prefilled with a catalyst and/or preferably while feeding supercritical CO2 or other fluids and/or other reactants in gaseous form, or by contacting such purified ambradiol and/or sclareolide with HTW within a pressure-controlled vessel, such vessel being optionally prefilled with a solid catalyst and/or preferably while feeding supercritical CO₂ or other fluids and/or other reactants in gaseous form such as dihydrogen, preferably in a counter-current manner, in said vessel.

Among extraction methods used in purification of products and performed within a pressured vessel, supercritical extraction presents several advantages that place it as a valuable alternative. This technique uses a supercritical fluid, which in certain conditions of pressure and temperature above critical point, may be considered as a solvent. Sometimes, the supercritical fluid is coupled with a co-solvent in order to adjust its polarity to that of the targeted compound(s). At the end of the extraction, the pressure is lowered and the state of the fluid converts from supercritical to gas, allowing its easy elimination from the extract.

Supercritical fluids present the advantage of exhibiting a liquid-like density, providing a high solvent power, and a gas-like viscosity and diffusivity, enhancing transport properties. Supercritical fluids can penetrate into porous or fibrous solids. It is the combination of all these properties that makes supercritical fluids particularly suitable for extractions and purifications. Of all eligible supercritical solvents, carbon dioxide is the supercritical fluid of choice for industrial applications due to mild critical temperature (31.1°C) and pressure (73.8 bar) of its critical point. Moreover, carbon dioxide is chemically inert, relatively nontoxic, odorless, noninflammable and of low cost. Also, the generally low polarity of carbon dioxide at supercritical conditions makes it a good solvent for the extraction of substances of low polarity and of low molecular weight (preferably below 1000 g.mol-1) (Darani K. and Mozafari M., J. Biochem. Tech., 2009, 2(1), 144-152), while high polarity and/or high molecular weight compounds remain generally insoluble in supercritical CO₂.

Supercritical fluids demand important initial investments in terms of infrastructure that may discourage using this method, all the more so when alternative techniques such as liquid-liquid extraction using organic liquid solvents may be used. However, supercritical CO₂ offers many advantages over conventional organic solvents such as achieving higher purity extracts generally free of polar or high molecular weight substances, no residual solvents, single-step processing, reduced operating costs, selective fractionation or faster separation. In addition, the oxygen free operating system prevents oxidation and the low temperatures used minimize thermal degradation of sensitive materials.

Therefore, the Applicant carried out research for applying supercritical fluids to extraction, purification and, generally, production of sclareolide, ambradiol and/or (-)-ambrafuran and, surprisingly, showed that sclareolide, ambradiol and (-)-ambrafuran are soluble in supercritical CO₂ without cosolvent.

Generally, the ability to extract and purify substances using supercritical CO₂ is difficult to predict without experimentation. The efficiency of supercritical extraction and purification depends altogether on the characteristics of the targeted substance such as polarity and molecular mass, on the temperature and pressure parameters of the supercritical fluid, but also on the characteristics of the medium containing the substances to be extracted, such as composition, polarity, granularity, etc. In weak solubility or affinity conditions, the addition of a co-solvent such as methanol or ethanol is often necessary but results in lower selectivity. Therefore, the ability to extract substances in high purity from a medium using supercritical fluids is to be explored on a case-by-case basis and conditions of pressure and temperature have to be optimized for each substance or mix thereof.

Whether or not sclareolide, (-)-ambrafuran and mostly ambradiol were soluble in pure supercritical carbon dioxide was therefore not foreseeable; to the contrary, in view of the polarity brought by the two hydroxyl functions in ambradiol and of the presence of oxygen atoms in sclareolide and in (-)-ambrafuran, the skilled artisan was induced to estimate that solubility of these compounds in supercritical carbon dioxide was mostly questionable.

Furthermore, the ability to extract sclareolide or ambradiol in high purity from a bioconversion broth required practical experimentation in order to test the relative affinity of the targeted substances for supercritical CO₂ versus their aqueous broth medium or the potential pollution of the supercritical extracts by additives contained in the broth such as growth factors, nutrients, substrates or surfactants or by by-products produced by the organism.

Tests were therefore carried out by the Applicant, and it was found that the extraction of sclareolide or ambradiol by supercritical CO₂ from the aqueous medium used for the biological conversion was possible, even in the presence of several additives such as growth factors, nutrients, substrates and surfactants, all of which could have retained ambradiol or sclareolide within the broth or polluted the extract.

The Applicant further found that several of the compounds used in yeast nutrient mediums and useful to promote yeast growth and, more specifically, to promote the growth of the organisms identified for the transformation of sclareol into the substances of interest, namely sclareolide and/or ambradiol, are not soluble in supercritical CO₂ and/or that their affinity for supercritical CO₂ did not allow their efficient extraction from the bioconversion broth impacting the purity of the recovered compounds of interest. Some of the compounds identified by the Applicant as potential growth promoters for the organisms as well as for yeasts in general and associated with low solubility in supercritical CO₂ include without being limited to: carbon sources such as glucose, galactose, L-sorbose, maltose, sucrose, cellobiose, trehalose, L-arabinose, L-rhamnose, ethanol, glycerol, L-erythrithol, D-mannitol, lactose, melibiose, raffinose, melezitose, starch, D-xylose, D-sorbitol, a-methyl-D-glucoside, lactic acid, citric acid, succinic acid; organic sources of nitrogen such as peptone, meat extract, yeast extract, corn steep liquor, casein, urea, amino acids but preferably inorganic sources of nitrogen such as: nitrates, nitrites, inorganic ammonium salts; inorganic salts such as phosphates of magnesium, potassium, calcium or sodium; several vitamins but preferably vitamin B1, B2, B3, B5, B6, B7, B9, B12; minerals such as Fe, Mo, Cu, Mn and B as well as most acids, preferably inorganic acids, used to adjust broth pH. The Applicant found that, generally, yeast growth promoters showed poor solubility in supercritical CO₂.

The Applicant further found that emulsifying compounds such as polysorbates and more specifically such as TWEEN 80, which are useful to improve the solubility in a fermentation broth of compounds otherwise hardly soluble in aqueous mediums such as sclareol, were not extracted from the fermentation broth at the temperatures and pressures needed to extract the compounds of interest. Moreover, the presence of such surfactants in the fermentation broth did not prevent the extraction of the compounds of interest using supercritical CO₂.

Therefore, the Applicant established that the product of a biological conversion performed using well-chosen growth factors and/or nutrients and/or emulsifiers may be purified, whenever needed, through supercritical extraction.

More generally, the Applicant found that sclareolide, ambradiol and (-)-ambrafuran were soluble in supercritical CO₂ in all of the operating conditions required for the production of sclareolide, ambradiol and (-)-ambrafuran.

According to a first embodiment, the supercritical fluid used in the supercritical extraction of the product of biological conversion of sclareol is supercritical carbon dioxide, preferably without but potentially with ethanol as a co-solvent.

According to one embodiment, the supercritical CO₂ extraction is carried at a temperature ranging from 31°C to 90°C, but preferably between 31°C and 45°C.

According to one embodiment, the supercritical CO₂ extraction is carried with a pressure ranging from 74 bars to 250 bars, but preferably between 80 bars and 150 bars.

According to one embodiment, the purity of sclareolide or ambradiol after supercritical CO₂ extraction ranges from 75% to 99%.

According to one embodiment, the yield of ambradiol or sclareolide after supercritical CO₂ extraction ranges from 85% to 99%.

The sclareolide-charged or ambradiol-charged supercritical CO₂ obtained at the end of the purification step may be passed in a separator vessel to recover purified sclareolide or ambradiol from the supercritical fluid following decompression or heating of the fluid to decrease its density and therefore release the organic substrate. According to another embodiment, sclareolide-charged or ambradiol-charged supercritical CO₂ obtained at the end of the purification step is directly used in a next conversion step.

### Hydrogenation of sclareolide into ambradiol

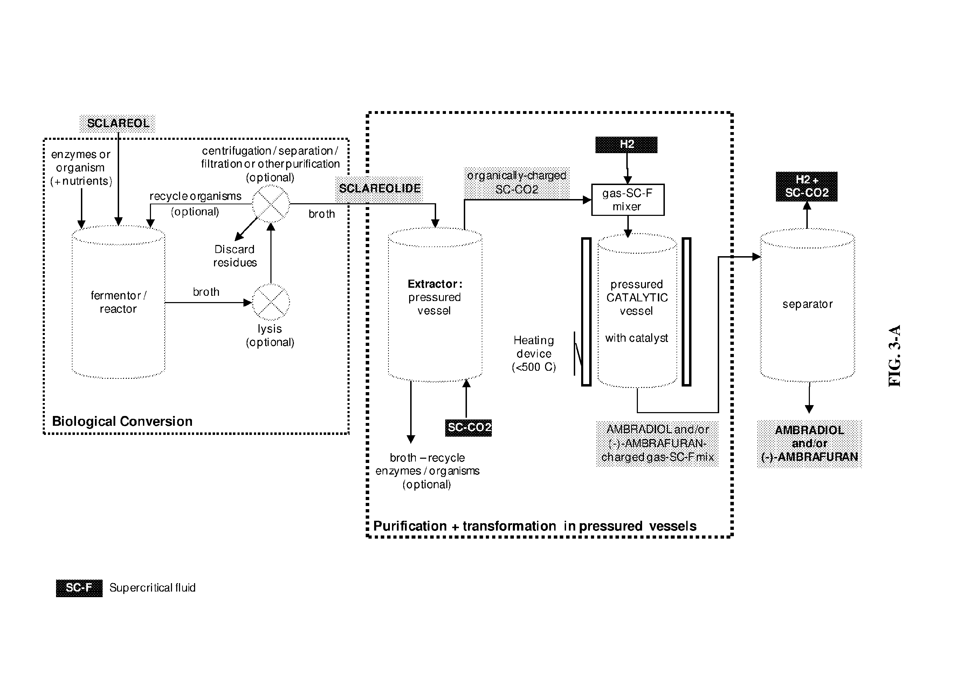

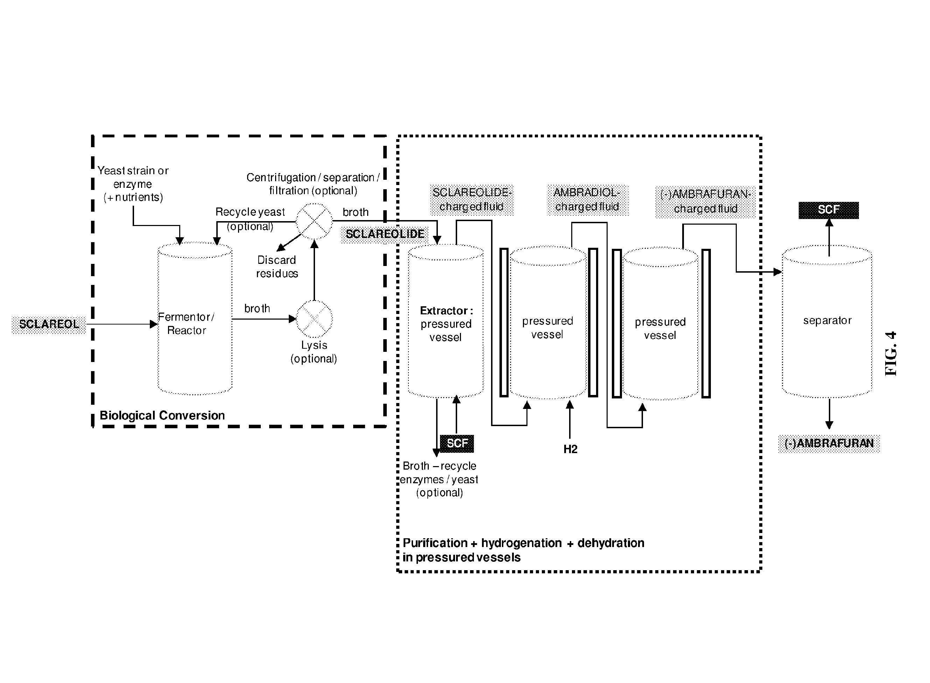

Ambradiol may be obtained through the biological conversion of sclareol in the presence of a suitable microorganism or, as shown in Fig.3-A and Fig. 4, through the biological conversion of sclareol into sclareolide in the presence of a suitable microorganism followed by, as it was found by the Applicant, the hydrogenation of sclareolide using H₂ in the presence of a metal catalyst. Although the former method seems superior to the latter, given that sclareolide is a substance with significant market interest used as a flavour in tobacco or food products and as a fragrance in detergents and cosmetics, it is an object of this invention to provide a method for producing both sclareolide and ambradiol (as an intermediary to (-)-ambrafuran) in a continuous integrated production process. Also, the pathway consisting in producing sclareolide from the bioconversion of sclareol and proceeding thereafter with the catalytic hydogenation of sclareolide into ambradiol constitutes an interesting alternative justified, if any, by economic considerations.

However, the catalytic conversion of sclareolide into ambradiol using a metal catalyst raises several issues.

One issue concerns the uncertainty over the stability of sclareolide and ambradiol when subjected to the high temperatures required for catalytic hydrogenations.

Also, sclareolide and ambradiol consist of white solids, which raises practical issues as per their introduction as solids in a continuous catalytic system, their capacity to flow within the system as defined by their viscosity and/or surface tension at operating conditions, or their capacity to be brought to a single or dual phase within the system.

More specifically, one issue concerns the introduction of a solid in a continuous pressurized catalytic transformation system. The review "Heterogeneous Catalytic Hydrogenation in Supercritical Fluids: Potential and Limitations" from Seki, Grunwaldt and Baiker lists 18 publications documenting hydrogenations performed in continuous flow reactors. None of the transformations concerns a solid at ambient temperature, except in the case of levulinic acid, which is solubilized in water prior to its introduction in the system developed by Bourne et al. The problem with sclareolide and ambradiol is that they are not soluble in water at room temperature. Although solvents such as ethanol or other organic solvents could be used, these could interfere with the catalytic hydrogenation, contaminate the substrate with by-products that would be in turn difficult to separate from the substrate.

Another issue concerns the dynamics of catalytic transformations in biphasic systems whereby the reactants, in our case H₂ and sclareolide, do not consist of the same phase, therefore causing mass transport problems. When the substrate is a liquid soluble in a supercritical fluid, the system is usually either brought back to a single phase by solubilizing the substrate in the supercritical fluid in order to create a single phase with gazeous dihydrogen, therefore reducing mass transport problems, or the supercritical fluid is kept at a lower pressure than required to maintain a single phase system, thereby resulting in a biphasic expanded liquid system where the liquid substrate, swollen by the supercritical fluid, still retains a high capacity to dissolve gaseous hydrogen, again reducing mass transport problems. In the first case, given that hydrogenations are mostly performed at elevated temperatures above 100°C or 140°C, the equipment and energy costs required to maintain the high supercritical fluid densities necessary to create a single phase may make the solution economically not viable. In the second case, maintaining a biphasic system with an expanded liquid requires that the substrate be liquid at the required operating conditions.

A further issue concerns the flow of substate and transformation product within the catalytic system. When low supercritical fluid densities compatible with expanded liquid biphasic systems are used, the substrate or its transformation product are not carried by the supercritical fluid. Therefore, in order to ensure substrate and product circulation within the catalytic system, it is necessary that both substrate and transformation product be either liquid with an appropriate viscosity and/or surface tension or dissolved in an appropriate solvent, lest they remain in the system and/or clog the catalyst.

The above issues justify why current work in catalytic hydrogenations in supercritical fluids has been restricted to liquids or substances soluble in a liquid solvent compatible with the envisaged reaction.

With respect to all the issues raised above, the Applicant has found that a supercritical fluid such as supercritical CO₂ can be used as a solvent to solubilize sclareolide around room temperature in order to introduce it in the catalytic system.

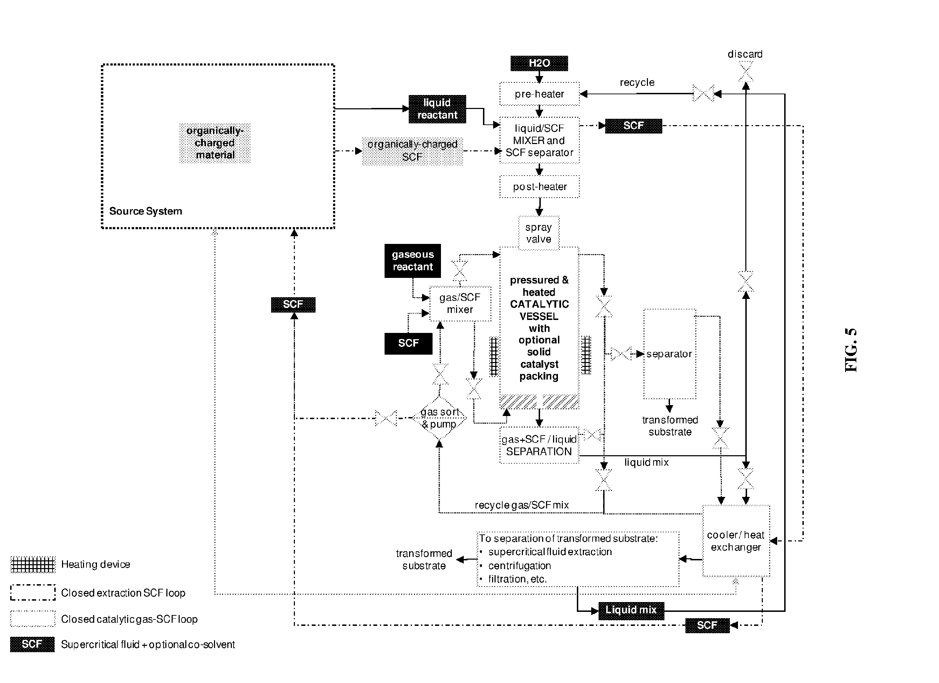

Alternatively, as shown in Fig.5, knowing that the melting point of sclareolide is around 125°C, the Applicant has found that solids, such as sclareolide, that are soluble in a supercritical fluid, can further be, depending on operating conditions, solubilized in or finely mixed with HTW prior to entry into the catalytic vessel by contacting the organically-charged supercritical fluid with HTW in a separate vessel, therefore causing the supercritical fluid to expand and release its organic charge in the HTW medium. This step allows the organically-charged supercritical fluid to be further recycled into its source system (for example, the extraction/purification system) with no need to depressurize the fluid and avoiding any pollution of this fluid by products of the catalytic process. This step also allows bringing in a short time a substrate such as sclareolide to a temperature where it is in liquid form and to further solubilize or finely mix it with HTW in order to allow for its continuous introduction into the catalytic vessel and facilitate its transformation within the system.

The Applicant has also found that, at the elevated temperatures required for catalytic hydrogenation using a metal catalyst, sclareolide and its hydrogenated product ambradiol are in liquid form and can be brought to expanded liquid conditions within the catalytic vessel. The Applicant has also found that expanded liquid conditions not only reduced mass transports problems, which resulted in higher conversion rates, but also improved the viscosity and reduced surface tension of sclareolide and ambradiol, therefore improving their flow within the system. The Applicant believes that the expanded liquid properties of sclareolide and ambradiol at operating conditions limit surface tension and reduce the viscosity of liquid sclareolide and/or liquid ambradiol, thereby favouring the progress of the liquid through gravity toward the bottom of the catalytic vessel. Additionally, introducing sclareolide as a mixture with HTW contributed to improving further the progress of the substrate within the system without reducing conversion rates.

Furthermore, the Applicant surprisingly obtained good results when carrying the hydrogenation of sclareolide into ambradiol in the presence of dihydrogen using catalysts made of metals selected from group 7B, 8, 9, 10 or 11 of the Periodic Table or mix thereof, preferably consisting of a mix of Cu and Pd on KOH support or of Cu on Si support or of CuOx on SiOx support, under single phase as well as biphasic conditions.

The Applicant has found that mixing sclareolide with HTW afforded good catalytic conversion rates.

Moreover, it was found that sclareolide and ambradiol have a good stability to the heat and pressure necessary to conduct such hydrogenations.

Finally, the Applicant found that, at operating conditions, liquid ambradiol would easily flow down the catalytic system and could be collected from inside a separation vessel where products flowing from the catalytic vessel are collected.

The Applicant found that the gaseous/supercritical mix containing dihydrogen and supercritical CO₂ could be recycled from the catalytic separation vessel back to the catalytic vessel with no need to depressurize or change its temperature.

The Applicant believes that such method and associated catalytic device could be used for the catalytic transformation of a large number of substances which are solids and soluble in supercritical fluids at ambient temperature, such as but not limited to mono-, sesqui-, di- and triterpenes and would be in liquid form within the catalytic device at the temperature conditions required for the contemplated catalytic transformation.

Using the transformation process described above results in:
- achieving a continuous hydrogenation of sclareolide into ambradiol with high yield and high purity;
- allowing for the continuous processing of solids at ambient temperature using supercritical fluids, HTW and reagents such as H2;
- avoiding the use of organic solvents and waste-producing or toxic reagents;
- allowing for the recycling of the sclareolide-charged supercritical fluid back to the extraction-purification system with no change in pressure and no contamination from substances present in the catalytic system;
- allowing for the recycling of the gaseous and supercritical mix within the catalytic system with no need to separate supercritical fluids from gaseous reagents or to depressurize supercritical fluids to recover transformed products.

Therefore, according to one embodiment, ambradiol may be synthesized through catalytic hydrogenation by feeding H₂, sclareolide in HTW and supercritical CO₂ into a pressured vessel packed with a metal catalyst.

According to a preferred embodiment, the sclareolide is brought to the catalytic system by supercritical CO₂, which is brought to flow through a first mixer system where it contacts HTW and releases its sclareolide content into the HTW before being recycled to the extraction/purification system. At this stage, the sclareolide is in liquid form and, depending on operating conditions, may be either solubilized in HTW or in colloidal form inside HTW.

According to another embodiment, sclareolide is not released into HTW and flows through the catalytic system carried by its original supercritical CO₂.

According to an embodiment, the catalytic vessel is coupled with a heating device.

According to an embodiment, the supercritical fluid is brought to a pressure within the catalytic vessel such as to achieve a single phase with liquid sclareolide.

According to a preferred embodiment, the supercritical fluid is maintained at a pressure within the device such as to achieve expanded liquid conditions with liquid sclareolide.

According to one embodiment, the pressure used in the hydrogenation step ranges from 10 to 650 bars, preferably from 80 to 250 bars. According to one embodiment, the temperature used in the hydrogenation step ranges from 50 to 400°C, preferably from 120 to 200°C.

According to one embodiment, the catalytic device is supplemented with another solvent fed from the top of the device in order to increase the velocity of the compounds within the device.

According to one embodiment, ambradiol is recovered from the catalytic system by expanding the ambradiol-rich supercritical fluid inside a separator vessel.

According to another embodiment, ambradiol is recovered from the catalytic system in an aqueous medium at the bottom of the catalytic separation vessel, ambradiol being further extracted from such medium by conventional methods such as filtration, centrifugation or separation but prefereably using a supercritical fluid.

Conditions used for the catalytic hydrogenation of sclareolide into ambradiol in supercritical CO₂ are compatible with a continuous process and do not require the use of consumable chemicals other than dihydrogen.

### Dehydration of ambradiol into (-)-ambrafuran using solid desiccants

It was found by the Applicant, as shown in Fig.3-B and Fig.4, that the good diffusivity of supercritical fluids in porous mediums was of great interest for the diffusion of ambradiol into solid desiccants such as molecular sieves or, more specifically, into activated zeolites and for the further recovery of (-)-ambrafuran from such desiccants after dehydration of ambradiol.

The Applicant found that supercritical fluids could also be used for the continuous desorption of water previously adsorbed by solid desiccants such as zeolites during the dehydration of ambradiol.

The Applicant also found that the dehydration of ambradiol into (-)-ambrafuran using solid desiccants such as zeolites could be performed simultaneously with the dehydration of such desiccants using the same flow of supercritical fluid without noticeable rehydration of (-)-ambrafuran.

This finding led to improved results in comparison with the disclosure of patent application US2010/0248316. The Applicant also found conditions in which (-)-ambrafuran may be selectively recovered from zeolite, without obstructing its porous channels.

The use of activated zeolite and, more generally, of solid desiccants such as molecular sieves, is known to be constraining due to their quick deactivation from the adsorption of water. However, the Applicant hereby discloses a device wherein a solid desiccant is closely packed within an airtight column compatible with supercritical extraction, and implements, in an embodiment of this invention, a process with this device, wherein ambradiol dissolved in supercritical CO₂ may be passed in the column with a controlled flow, resulting in total conversion of ambradiol into (-)-ambrafuran, the latter being further extracted from the column by the same flow of supercritical CO₂, without production of by-products. At the same time, the flow of supercritical CO₂ serves to carry away water produced during the dehydration of ambradiol and adsorbed onto the desiccant, thereby preventing early deactivation of such desiccant, without any noticeable rehydration of the dehydrated product.

According to one embodiment, (-)-ambrafuran is synthesized from purified ambradiol obtained from the first step of the process of the invention - i.e. biological conversion - by feeding ambradiol-charged supercritical CO₂ into a pressured vessel filled with activated zeolite and optionally coupled with a heating device.

According to one embodiment, the (-)-ambrafuran-charged supercritical CO₂ obtained at the end of the dehydration step may be passed in a separator vessel to recover purified (-)-ambrafuran from the supercritical fluid.

According to one embodiment, the pressure used in the above dehydration ranges from 73 bars to 500 bars, preferably from 80 bars to 150 bars. According to one embodiment, the temperature used in the above dehydration ranges from 0°C to 350°C, preferably from 50°C to 250°C. According to one embodiment, a CBV320A from Zeolyst Intl zeolite is used in the dehydration step; according to other embodiments, a LaHY-catalyst, a CaHY-catalyst, a H-ZSM-5 zeolite catalyst or a montmorillonite catalyst is used.

Combining solid desiccant-catalyzed dehydration and supercritical conditions contributes to:
- maximizing reactivity by improving contact between ambradiol and the solid desiccant;
- improving reaction rates as supercritical CO₂ above 100 bars results in the formation of carboxylic acids that will promote the dehydration reaction;
- preventing zeolite discharge from contact with outside air humidity through confinement within air-tight pressured vessel;
- facilitating separation between dehydrating agent and organically-charged CO₂, therefore avoiding centrifugation or filtration and further liquid-liquid purifications and solvent-drying steps.

The Applicant also found conditions for reactivating solid desiccants without any need to discharge the column, i.e. in a continuous or at least a semi-continuous process. According to one embodiment, a vacuum pump is added to the pressured vessel filled with zeolite. In this embodiment, the use of the vacuum pump and of the heating device allows recharging of the catalyst without off-loading the vessel, in a semi-continuous process.

According to one embodiment, the flow of organically-charged supercritical CO₂ is stopped punctually to heat the vessel up to 500°C under vacuum, thereby recharging the desiccant.

According to another embodiment, the flow of organically-charged supercritical CO₂ is punctually stopped and replaced with a flow of pure supercritical CO₂, preferably at temperatures above 150°C, this flow drying the solid desiccant.

According to another embodiment, the flow of organically-charged supercritical CO₂ is not interrupted for zeolite drying. In this embodiment, ambradiol dehydration is continuously performed at a temperature over 20°C, preferably over 80°C, more preferably over 150°C. In this case, the dehydration of ambradiol is a continuous process, with no need to recharge or dry the solid desiccant separately from the dehydration reaction.

Therefore, conditions used for the dehydration of ambradiol into (-)-ambrafuran in supercritical CO₂ are compatible with a continuous or semi-continuous process and do not require the use of consumable chemicals.

### Synthesis of (-)-ambrafuran in subcritical water

The Applicant also investigated, as shown in Fig.3-C, the feasibility of the conversion of sclareolide or ambradiol into (-)-ambrafuran in a pressure-controlled and heated vessel filled with HTW, optionally mixed with a catalyst and optionally fed with supercritical CO₂ and/or gases such as H2.

It was previously shown that the dehydration of 1,4-butanediol into tetrahydrofuran may be carried out in HTW (Richter T. and Vogel H., Chem. Eng. Technol., 2001, 24, 340-343; Hunter S., Ehrenberger C. and Savage P., J. Org. Chem., 2006, 71, 6229-6239). Moreover, in these conditions, supercritical CO₂ displays acid-catalyst properties. However, these conditions were never envisaged for the synthesis of (-)-ambrafuran from sclareolide or ambradiol. Indeed, catalytic mechanisms are difficult to predict and the resistance of reactants and products might have prevented reaching the temperatures needed to operate reactions of hydrogenation and/or dehydration.

The use of HTW altogether as a reaction medium, a solvent and a catalyst in dehydration transformations is documented in JOC Article "Kinetics and Mechanism of Tetrahydrofuran Synthesis via 1,4-Butanediol Dehydration in High-Temperature Water", where Hunter, Ehrenberger and Savage describe the dehydration of the diol 1,4-butanediol (BDO) to the furan tetrahydrofuran (THF) using sub-critical water (HTW) at temperatures between 200°C and 350°C. However, according to Applicant's knowledge, no specific information can be found in prior art regarding the transformation of sclareolide or ambradiol using HTW.

Due to the configuration of the ambradiol molecule, one skilled in the art, in view of the above article, using HTW on ambradiol could lead to numerous possible transformations. Also, at ambient temperature, BDO and THF, are liquids miscible with water, whereas sclareolide and ambradiol consist of white solids immiscible with water. No indication was found in the prior art as regards the ability of sclareolide or ambradiol to form a unique phase with HTW, the impact of HTW on such molecules at a given temperature as well as the very stability of such molecules at the temperatures potentially leading to the envisaged transformations. Finally, no information was found on hydrogenation of the sclareolide intermediate or, as a proxy, γ-butyrolactone (GBL), into respectively ambradiol and BDO using HTW.

The Applicant however carried tests using a pressure-controlled vessel containing HTW, and noticed that ambradiol could be dehydrated into ambrafuran using HTW, preferably with a catalyst such as CO2 or silicate or other catalysts and, more surprisingly, to the isomeric conformation of interest, (-)-ambrafuran.

The Applicant also carried tests using a pressure-controlled vessel containing HTW, and noticed that sclareolide could be transformed into ambrafuran using HTW, preferably with a catalyst such as CO2 or silicate or Zn or other catalysts and, more surprisingly, to the isomeric conformation of interest, (-)-ambrafuran.

It was also found by the Applicant that (-)-ambrafuran may be synthesized from purified or not-purified ambradiol as well as from purified or non-purified sclareolide.

The Applicant also found a method for, altogether, recycling the supercritical fluid carrying untransformed sclareolide or ambradiol with no required change of pressure and recycling the gaseous and supercritical mix used within the HTW vessel with no required change of pressure. Such objective is achieved, as shown in Fig.3-C, by feeding the sclareolide- or ambradiol-charged supercritical fluid into a pressured "Mixer/Separator" vessel fed with HTW. As the supercritical fluid expands, it releases its organic content in the HTW, which is then passed onto the pressure-controlled catalytic HTW vessel. The supercritical fluid carrying sclareolide or ambradiol can then be recycled into its source system free of any substantial organic charge. The catalytic vessel containing organically-charged HTW can then be optionally fed countercurrently with a continuously-recycled mix containing supercritical fluids, preferably CO2, and/or other gaseous reactants, preferably H2, and/or be optionally filled with a catalyst, potentially Zn. The gaseous and supercritical fluid mix fed to the HTW catalytic vessel can then be recycled into the same vessel with no required change of pressure. Following conversion, the HTW is transferred into a cooler before being extracted by conventional means including centrifugation or filtration but preferably using a supercritical fluid.

According to one embodiment, purified sclareolide or ambradiol carried by supercritical CO2 is fed into the Mixer/Separator.

According to one embodiment, purified sclareolide or ambradiol carried by supercritical CO2 is fed directly into the pressure-controlled vessel containing HTW.

According to another embodiment, the fermentation broth resulting from the biological conversion step and containing sclareolide or ambradiol is used to directly synthesize (-)-ambrafuran by feeding it into a catalytic vessel at HTW conditions.

In one embodiment, the catalytic vessel containing organically-charged HTW is fed countercurrently with a continuously-recycled mix containing supercritical CO2 and/or other gaseous reactants such as H2 and/or be optionally mixed with a catalyst.

In one embodiment, following conversion, the HTW is transferred into a cooling device before being extracted by conventional means including centrifugation or filtration but preferably using a supercritical fluid.

According to one embodiment, the pressure used in the synthesis of (-)-ambrafuran is ranging from 1 to 750 bars; when using supercritical CO2 preferably ranging from 100 bars to 250 bars.

According to one embodiment, the temperature used in the direct synthesis of (-)-ambrafuran is ranging from 120 to 550°C, preferably ranging from 250°C to 350°C.

According to one embodiment, when using ambradiol to produce (-)-ambrafuran, the vessel used in the above conversion is optionally packed with a solid acid catalyst such as silica.

According to one embodiment, when using sclareolide to produce (-)-ambrafuran, the vessel used in the above conversion is optionally packed with a solid catalyst such as Zn.

The advantage of this direct synthesis of (-)-ambrafuran is that conditions are compatible with a continuous process and without the use of consumable chemicals other than HTW. Another advantage is that these conditions improve the reaction rate, especially in the case of ambradiol dehydration thanks to the acid catalyst properties of supercritical CO₂.

### Hydrogenation-Dehydration device

Several catalytic devices have been reported in prior art. WO 02/081414 reports a novel hydrogenation device compatible with heterogeneous catalysts.

Given that the transformations needed for the production of (-)-ambrafuran from sclareolide or ambradiol include a hydrogenation step as well as a dehydration step, that the type of catalyses include single phase and/or biphasic reactions, performed on solid and/or HTW catalysts using supercritical fluids or gaseous reactants, there was therefore a need for a device capable of performing all such reactions. In that respect, WO 02/081414 did not offer the ability to use HTW as catalytic medium as, among other missing features, it did not allow the gaseous and supercritical mix to flow countercurrently. Also, the device did not allow for the creation of a gaseous/supercritical fluid mix loop restricted to the catalytic vessel, thereby avoiding unnecessary changes of pressure.

The Applicant has a catalytic system, as shown in Fig.5, that allows for all the hydrogenation and dehydration transformations involved in the production of (-)-ambrafuran and documented in the above sections and generally allows for hydrogenations and/or dehydrations to be performed using a wide range of reactants, mediums and catalysts.

More generally, among other functionalities, the catalytic system described allows for:
- The recycling of the organically-charged supercritical fluid into the extraction-purification system without decompression or pollution from the catalytic system;
- The handling of liquid, gaseous or solid substrates that can be further transferred to an HTW medium prior to entry in the catalytic vessel;
- Using within the catalytic vessel a gaseous and supercritical mix that can be recycled within the vessel without change of pressure;
- Implementing single-phase catalyses as well as Biphasic catalyses;
- Using gaseous mediums as well as supercritical mediums;
- Implementing a top-down flow for substrates and for HTW;
- Implementing a top-down flow as well as a bottom-up flow for gaseous and supercritical fluids, each one being respectively useful in the case supercritical single-phase and biphasic catalyses in gaseous or supercritical environment are implemented and in the case catalysis in HTW medium is implemented.

### Preferred embodiment

A preferred embodiment of the invention is the following:
in a first step of the process of the present invention, sclareolide or ambradiol, produced by biological conversion of sclareol are extracted by supercritical CO₂ extraction from the nutrient medium used for cultivating the microorganism. The supercritical fluid containing sclareolide may then directly be used for the hydrogenation, for the production of ambradiol. The supercritical fluid containing ambradiol, either coming from the hydrogenation of sclareolide or from the biological conversion of sclareol, may then directly be used in a step of dehydration to synthesize (-)-ambrafuran. Advantageously, the dehydration may be catalyzed by the use of zeolites and the close contact between ambradiol and catalyst zeolite is obtained by the used of supercritical CO₂. Dehydration to (-)-ambrafuran may also be conducted by reacting ambradiol in subcritical water at high temperature, optionally using a catalyst. It was also found that both hydrogenation and dehydration were possible in high temperature water, in a pressured vessel, also using supercritical CO₂. Moreover, it was discovered that this reaction may occur without previous purification of sclareolide and ambradiol.

### EXAMPLES

The present invention is further illustrated by the following examples.

### Example 1:

Extraction of sclareolide, ambradiol and ambrafuran from an aqueous medium.

1g of sclareolide, 1 g of ambradiol and 1 g of ambrafuran were each mixed with 100 ml of water and further subjected to supercritical extraction using pure CO2 at 140 bars and 35°C. In each case, after 30 min, over 98% of the substances contained in the extractor had been transferred to the separator vessel.

### Example 2:

Conversion of ambradiol to (-)-ambrafuran using HTW.

1 g of ambradiol was injected into 100 ml of HTW contained in a pressure-controlled vessel using supercritical CO2. The vessel containing HTW was pressurized at 200 bars using CO2 and contained silica as a solid acid catalyst. The temperature within the vessel was kept constant at 250°C. After 30 minutes, the organic extract contained in the water was analyzed by GC. Over 80% of the extract consisted of (-)-ambrafuran.

### Example 3:

Conversion of ambradiol to (-)-ambrafuran using a zeolite.

100 g of zeolite CBV320A was purchased from zeolyst Intl. The zeolite was dried during 30 min at 500°C in an airtight vessel under vacuum. 5g of ambradiol was then injected into the vessel using supercritical CO2 at 150 bars. After 2 hours, the zeolite was subjected to a supercritical extraction using pure CO2. The substance recovered was analyzed by GC, providing over 98% of (-)-ambrafuran.

## Claims

1. Process for manufacturing products of interest selected from the group comprising ambradiol, sclareolide and (-)-ambrafuran, from a starting material which is sclareol, said process comprising a step of biological conversion of sclareol and at least one further step performed within a pressured vessel.

2. Process according to claim **1,** where the further step is a purification step using CO₂ supercritical extraction, or a further chemical conversion step which is selected from the group comprising supercritical hydrogenation, supercritical dehydration, hydrogenation in subcritical water or dehydration in subcritical water.

3. Process according to claim **1** or claim **2,** wherein the process is continuous or semi-continuous.

4. Process according to anyone of claims **1** to **3,** wherein the pressured vessel is coupled to a heating device and/or equipped with a vacuum pump.

5. Process according to anyone of claims **1** to **4,** wherein the vessel is charged with a solid dessicant catalyst, which is preferably activated zeolite.

6. Process according to anyone of claims **1** to **5,** wherein in the step of biological conversion, sclareol is contacted with a microorganism capable of converting it into sclareolide or ambradiol, said microorganism being in an aqueous nutrient medium, and the further step performed within a pressured vessel is at least one chemical transformation, and/or a purification, of sclareolide or ambradiol.

7. Process according to claim **6,** wherein the microorganism is selected from the group comprising *Cryptococcus albidus* saito, *skinner var. albidus,* ATCC 20918 or *Cryptococcus albidus,* ATCC 20921, and is capable to convert sclareol into sclareolide.

8. Process according to claim **6** or claim **7,** wherein in the further step, sclareolide is chemically transformed into ambradiol through a hydrogenation reaction within a pressured vessel.

9. Process according to claim **6,** wherein the microorganism is selected from the group comprising *Hyphozyma roseoniger* (CBS214.83 and ATCC 20624) and is capable to convert sclareol into ambradiol.

10. Process according to anyone of claims **6** to **9,** wherein sclareolide and/or ambradiol are purified through supercritical CO₂ extraction followed, whenever necessary, by a separation step to recover purified scareolide or ambradiol from the supercritical fluid.

11. Process according to anyone of claims **6, 8** to **10,** wherein ambradiol is chemically transformed into (-)-ambrafuran through a dehydration step of ambradiol within a pressured vessel.

12. Process according to claim **11,** wherein the dehydration is catalyzed by a zeolite catalyst charged in the pressured vessel and the dehydration reaction is performed under supercritical conditions.

13. Process according to anyone of claims **1** to **12** where the supercritical extraction occurs at a pressure of 80 to 350 bars and at a temperature of 15 to 250 °C.

14. Device for implementing a process according to anyone of claims **1** to **13,** comprising a pressured vessel which may be packed with a catalyst, which preferably is activated zeolite, the pressured vessel being preferably associated with a heating device capable of heating the walls of the vessel.
